# EUROPEAN PATENT APPLICATION

(11) **EP 4 068 302 A1**
(43) Date of publication of application: **05.10.2022**
(21) Application number: 20891418.4
(22) Date of filing: 25.11.2020
(51) Int. Cl.: G16H 40/67

(54) **MEDICAL DATA MANAGEMENT METHOD, APPARATUS, SYSTEM AND SERVER**

(30) Priority: 25.11.2019 CN 201911167852
(71) Applicant: BOE Technology Group Co., Ltd., Beijing 100015 (CN)
(72) Inventor: GAO, Yuan, Beijing 100176 (CN); LIU, Zigang, Beijing 100176 (CN); WANG, Zhou, Beijing 100176 (CN); HUANG, Dongsheng, Beijing 100176 (CN); HAN, Yang, Beijing 100176 (CN); ZHOU, Li, Beijing 100176 (CN); LI, Xin, Beijing 100176 (CN)
(74) Representative: Brötz, Helmut
(86) International application number: PCT/CN2020/131575
(87) International publication number: WO 2021/104334

(57) **Abstract**

A medical data management method, a medical data management apparatus, a medical data management system, and a medical data management server. The medical data management method includes: receiving a processing scheme associated with a first client from a server; receiving medical data corresponding to the processing scheme from a medical device associated with the first client; and transmitting the medical data to the server.

## Description

### TECHNICAL FIELD

The present disclosure relates to the field of data processing, in particular to a medical data management method, a medical data management apparatus, a medical data management system, and a medical data management server.

### BACKGROUND

The chronic disease has become an important factor endangering people's health. Most users with the chronic disease need long-term medication, treatment, and self-management. Generally, many users with the chronic disease have poor compliance and may not persist for a long time in the process of self-management, which leads to the deterioration of the disease and the decline of the quality of life. In addition, in different stages of the development of the chronic disease, the users need to receive regular follow-up visits and/or recovery treatment in a general hospital or receive more professional treatment in a more specialized 3A grade hospital. In this process, the users need to select an appropriate hospital for treatment.

### SUMMARY

According to one aspect of the present disclosure, a medical data management method is provided. The method is executed by a first client, and includes: receiving a processing scheme associated with the first client from a server; receiving medical data corresponding to the processing scheme from a medical device associated with the first client; and transmitting the medical data to the server.

In some embodiments, the medical data management method further includes: determining a reminding message according to the received processing scheme; and outputting the reminding message.

In some embodiments, the medical data management method further includes: forwarding the reminding message to a third client associated with the first client.

In some embodiments, the medical device includes at least two of: a lung function instrument, a ventilator, an oxygen generator, a sleep screening instrument, an atomizer, and a non-invasive blood multi-parameter detector.

According to another aspect of the present disclosure, a medical data management method is also provided. The method is executed by a second client, and includes: receiving a processing scheme associated with a first client from a server, receiving medical data associated with the first client from a medical device; updating the processing scheme based on the received medical data, and transmitting the updated processing scheme to the server.

In some embodiments, the first client and the second client are associated with each other.

In some embodiments, the second client is a client of a first-level hospital, and the updated processing scheme includes: a processing scheme for leaving hospital, a processing scheme for staying in hospital, or a processing scheme for transferring to a hospital with higher level.

In some embodiments, the second client is a client of a second-level hospital, and the updated processing scheme includes: a processing scheme for transferring to a hospital with lower level; or a processing scheme for staying in hospital.

According to another aspect of the present disclosure, a medical data management method is also provided. The method is executed by a server, and includes: receiving medical data from a medical device from a first client, transmitting the medical data received from the first client to a second client, receiving an updated processing scheme from the second client, and transmitting the updated processing scheme to the first client.

According to another aspect of the present disclosure, a medical data management apparatus is also provided. The medical data management apparatus includes: an input unit, configured to receive a processing scheme associated with the medical data management apparatus from a server; a medical data receiving unit, configured to receive medical data corresponding to the processing scheme from a medical device associated with the medical data management apparatus; and an output unit, configured to transmit the medical data to the server.

In some embodiments, the medical data management apparatus further includes: a reminding unit, configured to determine a reminding message according to the received processing scheme. The output unit is further configured to output the reminding message.

In some embodiments, the medical data management apparatus further includes: a forwarding unit, configured to forward the reminding message to a third client associated with the medical data management apparatus.

In some embodiments, the medical device includes at least two of: a lung function instrument, a ventilator, an oxygen generator, a sleep screening instrument, an atomizer, and a non-invasive blood multi-parameter detector.

According to another aspect of the present disclosure, a medical data management apparatus is also provided. The medical data management apparatus includes: an input unit, configured to receive a processing scheme associated with a first client from a server, a medical data receiving unit, configured to receive medical data from a medical device associated with the medical data management apparatus, an updating unit, configured to update the processing scheme based on the received data, and an output unit, configured to transmit the updated processing scheme to the server.

In some embodiments, the first client and the medical data management apparatus are associated with each other.

In some embodiments, the medical data management apparatus is a client of a first-level hospital, and the updated processing scheme includes a processing scheme for leaving hospital, a processing scheme for staying in hospital, or a processing scheme for transferring to a hospital with higher level.

In some embodiments, the medical data management apparatus is a client of a second-level hospital, and the updated processing scheme comprises a processing scheme for transferring to a hospital with lower level, or a processing scheme for staying in hospital.

According to another aspect of the present disclosure, a medical data management server is also provided, and includes a receiving unit and a transmitting unit. The receiving unit is configured to receive medical data from a medical device from a first client, the transmitting unit is configured to transmit the medical data received from the first client to a second client, the receiving unit is further configured to receive, from the second client, an updated processing scheme based on the medical data, and the transmitting unit is further configured to transmit the updated processing scheme to the first client.

According to another aspect of the present disclosure, a medical data management system is also provided, and includes a first client, configured to receive medical data from a medical device; a second client, associated with the first client; and a server, configured to store a processing scheme associated with the first client and medical data, transmit the processing scheme and the medical data to the second client, receive an updated processing scheme from the second client, and transmit the updated processing scheme to the first client.

In some embodiments, the second client is a client of a first-level hospital or a client of a second-level hospital.

Using the medical data management method, medical data management apparatus, and system provided by the present disclosure, the first client associated with the user can receive a processing scheme for the current stage of illness of the user from the server, and can supervise the user to perform treatment by himself according to the processing scheme. In addition, the first client can also output a reminding message according to the received processing scheme, so that the user can be supervised effectively. The doctor can use the client to receive historical medical data and medical record of the user from the server, so as to determine the current physical condition of the user more accurately. In addition, in the case where the user needs to change a hospital, the doctor can propose a referral request for the user conveniently by using the client, thus improving the communication efficiency between different hospitals. Therefore, the efficiency of communication and information acquisition between the doctor and the user can be improved in the visit process.

### BRIEF DESCRIPTION OF THE DRAWINGS

In order to more clearly illustrate the technical schemes of the embodiments of the present disclosure, the drawings of the embodiments will be briefly described in the following. It is obvious that the described drawings below are only related to some embodiments of the disclosure, and for those skilled in the art, other drawings can be obtained according to these drawings without inventive faculty. The following drawings are not deliberately scaled to the actual size, and the purpose is to illustrate the main idea of the present disclosure.
Fig. 1 illustrates a schematic flow chart of a medical data management method according to the embodiments of the present disclosure;
Fig. 2 illustrates another schematic flow chart of a medical data management method according to the embodiments of the present disclosure;
Fig. 3 illustrates yet another schematic flow chart of a medical data management method according to the embodiments of the present disclosure;
Fig. 4 illustrates a schematic diagram of a medical data management system according to the embodiments of the present disclosure;
Fig. 5A illustrates a schematic block diagram of a first client according to the embodiments of the present disclosure;
Fig. 5B illustrates a schematic diagram of an exemplary usage process of a first client in a home scene;
Fig. 6A illustrates a schematic block diagram of a second client according to the embodiments of the present disclosure;
Fig. 6B illustrates a schematic diagram of an exemplary usage process of a second client in a first-level hospital scene;
Fig. 6C illustrates a schematic diagram of an exemplary usage process of a second client in a second-level hospital scene;
Fig. 7 illustrates a schematic block diagram of a server according to the embodiments of the present disclosure;
Fig. 8 illustrates a schematic flowchart of a medical data management method according to another embodiment of the present disclosure;

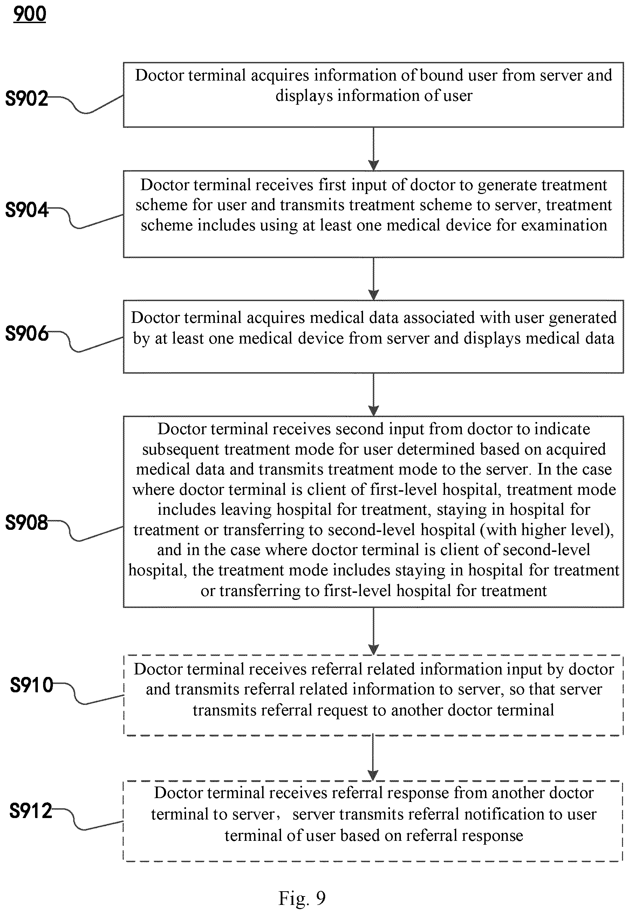

Fig. 9 illustrates another schematic flow chart of a medical data management method according to another embodiment of the present disclosure;

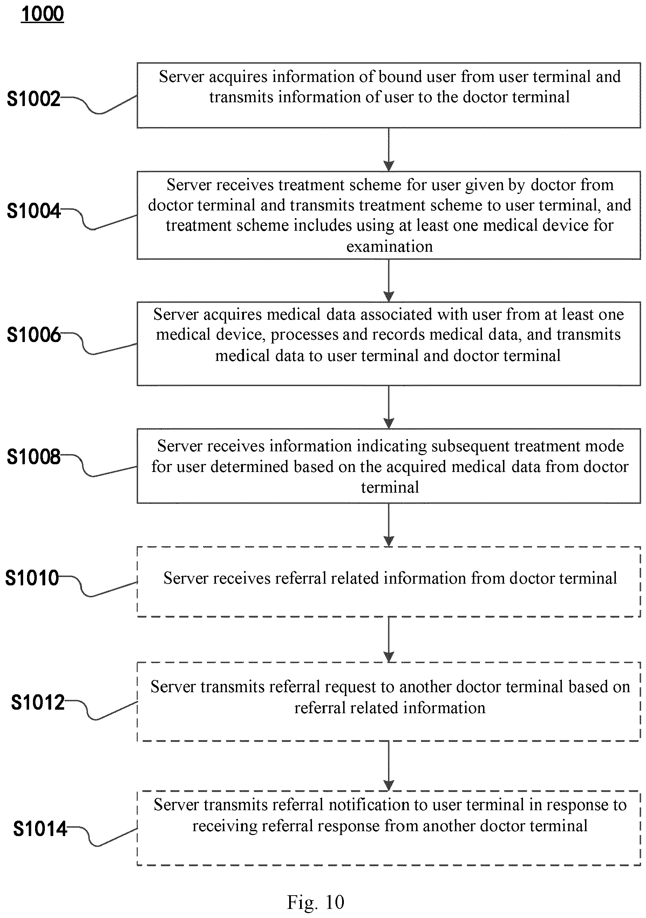

Fig. 10 illustrates yet another schematic flow chart of a medical data management method according to another embodiment of the present disclosure; and
Fig. 11 illustrates an architecture of a computing device according to the embodiments of the present disclosure.

### DETAILED DESCRIPTION

The technical schemes in the embodiments of the present disclosure will be described clearly and completely with reference to the drawings. Obviously, the described embodiments are only partial embodiments of the present disclosure, not all embodiments. Based on the embodiments of the present disclosure, all other embodiments obtained by ordinary technicians in the field without inventive faculty also belong to the protection scope of the present disclosure.

As illustrated in the present specification and claims, unless the context clearly indicates exceptional circumstances, the words "a," "an," "one," and/or "the" do not indicate the singular, but may also include the plural. Generally, the words "comprise/comprising" and "include/including" only imply that steps and elements that have been clearly identified are included, but these steps and elements do not constitute an exclusive list, and the method or apparatus may also include other steps or elements.

Although the present disclosure makes various references to some modules in the system according to the embodiments of the present disclosure, any number of different modules can be used and executed on the user terminal and/or server. The modules are illustrative only, and different modules may be used for different aspects of the system and method.

A flowchart is used in the present disclosure to illustrate the operations performed by the system according to the embodiments of the present disclosure. It should be understood that the preceding or following operations are not necessarily performed accurately in sequence. On the contrary, the various steps can be processed in a reverse order or at the same time as required. In addition, other operations can also be added to these processes, or one or several operations can be removed from these processes.

The chronic disease has become an important factor endangering people's health. Common chronic diseases include hypertension, hyperglycemia, chronic obstructive pulmonary disease, and others. It should be understood that the chronic disease generally includes a stationary stage and an acute stage. During the stationary stage, the user can take medicine/treatment regularly according to the doctor's advice without going to hospital. During the acute stage, the user needs to be examined in hospital and may need to stay in hospital for treatment.

Hereinafter, the principles of the present disclosure are described by taking the chronic obstructive pulmonary disease as an example. The chronic obstructive pulmonary disease (COPD) is a chronic airway disease characterized in persistent airflow restriction, which has a long course, easy to recur, and is the fourth most fatal disease in the world. It should be understood that without departing from the principles of the present disclosure, the method, apparatus, and system provided in the present disclosure can also be used for complications caused by the chronic obstructive pulmonary disease or any other diseases related to or unrelated to the chronic obstructive pulmonary disease.

Because of the lack of professional knowledge, many users are difficult to adhere to strict self-management during the stationary stage of the illness, which leads to the recurrence of the illness and the decline of the life quality. In the prevention, rehabilitation, and education part of the COPD, community health institutions may not give the users with the COPD much help because of the lack of staff and low enthusiasm. When the illness enters the acute stage, most users may choose to go to a 3A grade hospital for treatment. Even if the illness enters the stationary stage, the users are still unwilling to go to a community hospital for rehabilitation care. This is a waste of medical resources for the 3A grade hospital.

In order to solve the above problems, the present disclosure proposes a medical data management method and a medical data management system, the purposes of which are to effectively implement the data sharing between the user and doctors in different hospitals, and to help the user implement disease management more effectively.

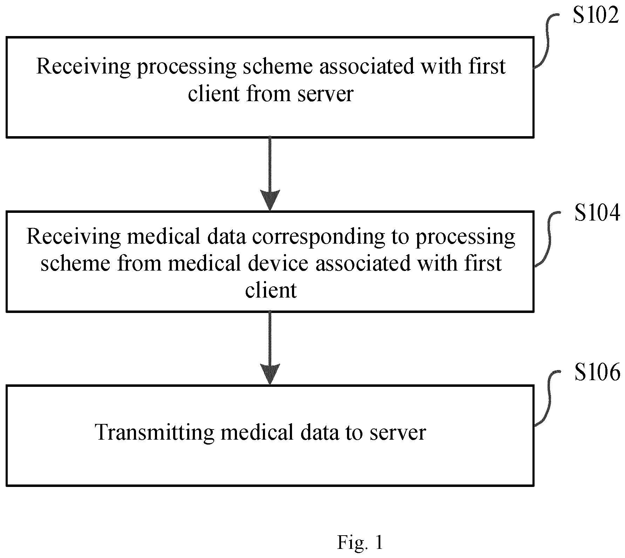

Fig. 1 illustrates a schematic flow chart of a medical data management method according to the present disclosure. The medical data management method illustrated in Fig. 1 may be performed by a first client. Hereinafter, the first client may refer to a client used by a user (i.e., a patient), also be referred to as a user terminal.

In step S102, a processing scheme associated with the first client may be received from a server. In some embodiments, the first client receives a user input and transmits the request for acquiring an associated processing scheme to the server to in response to the user input, thereby receiving the associated processing scheme. In other embodiments, the first client may transmit the request for acquiring the associated processing scheme to the server at a fixed period (e.g., every day, every week, every month, or any other predefined time period), so as to receive the associated processing scheme. In yet other embodiments, the first client may receive the processing scheme actively transmitted by the server.

In the case where the user uses the first client, the user can log in through a predefined user identification, so that the first client is associated with the user identification.

In some embodiments, the processing scheme may be a treatment scheme of the user corresponding to the first client.

In some implementations, the treatment scheme may be the medication scheme that indicates the user to take his own medicines at home. For example, the medication scheme may include the user using a medical device for the required treatment and/or examination at a predefined time. For example, the medical device may include a lung function instrument, a ventilator, an oxygen generator, an atomizer, a non-invasive blood multi-parameter detector, and a sleep screening instrument. Among them, the lung function instrument, the sleep screening instrument, and the non-invasive blood multi-parameter detector can be used to perform the required examination for the user. The ventilator, the oxygen generator, and the atomizer can be used to perform the required treatment for the user. In some examples, the medical device may include at least two of the above-mentioned medical devices. For example, the at least two medical devices may include at least one medical device for examination and at least one medical device for treatment.

In other implementations, the treatment scheme may indicate that the user should go to a hospital for treatment. For example, the treatment scheme may include the user going to a designated hospital regularly for reexamination. For another example, the treatment scheme may include the user going to a hospital for examination when the medical data of the user is abnormal.

In step S104, medical data corresponding to the processing scheme may be received from a medical device associated with the first client.

In some embodiments, the device such as the lung function instrument, ventilator, oxygen generator, atomizer, non-invasive blood multi-parameter detector, and sleep screening instrument can be associated with the user terminal, and the usage data of the device can be transmitted to the user terminal. For example, in the case where the user uses the medical device, the user terminal can receive the medical data generated by the medical device. In some examples, the user terminal may receive the medical data from the medical device through the wireless network such as Bluetooth or Wifi or any wired network.

In some embodiments, the user terminal may determine a reminding message according to the processing scheme received in step S102. According to the received treatment scheme, the user terminal can determine that the user should receive treatment through a specific medical device (for example, the atomizer) at a predefined time. In some examples, the reminding message for reminding the user to use the atomizer for treatment may be determined at a period of time before the predefined time (e.g., five minutes, ten minutes or other predefined time intervals before the predefined time), and the reminding message may be output. In other examples, if the user terminal does not receive the medical data from the atomizer at the predefined time, the reminding message for reminding the user to use the atomizer for treatment can be determined and then output. For example, if the user has not completed the specified treatment or examination behavior when a period of time (for example, one hour, two hours or other predefined time intervals) after the specified treatment time ends, the reminding message above can be output.

In some embodiments, the first client may transmit the message indicating that the specified behavior has been completed to the server so as to cancel the reminder. For example, the first client may transmit the received data from the medical device indicating that the user has completed the specified treatment or examination behavior to the server so as to indicate that the specified behavior has been completed. For another example, the first client may also transmit the picture (e.g., the photo and/or video about the user using the medical device) and/or text message indicating that the user has completed the specified treatment or examination behavior to the server, so as to indicate that the specified behavior has been completed.

In still other examples, the user terminal may also forward the reminding message to a third client associated with the first client. For example, the third client may be a client used by a relative/friend of the user associated with the first client. The user terminal can forward the reminding message to the third user terminal via the server.

In step S106, the first client may transmit the received medical data to the server. For example, the user terminal can transmit the received medical data to the server via any wireless network and wired network. The server can record the received medical data in the medical file associated with the user corresponding to the user terminal. The doctor can directly call the historical record of these pieces of medical data from the server through the client in the subsequent visit and treatment process of the user.

In some embodiments, the user can also use the first client to communicate with the doctor. For example, the user can transmit the description of his recent physical condition to the doctor through the first client. The doctor can view the user's description and give an answer, analysis, and visit reminder for the user's description through the doctor terminal. In this case, the user can receive the answer, analysis, and visit reminder given by the doctor through the first client. For example, the visit reminder may include the hospital recommended by the doctor.

In some embodiments, the first client may provide an assessment scheme to the user. For example, the user can fill in an assessment questionnaire for the life quality of the COPD user (CAT questionnaire) provided by the first client before going to the hospital for treatment. The first client can transmit the questionnaire filling result of the user to the client used by the doctor, and receive the assessment result made by the doctor according to the questionnaire filling result. The assessment result may include suggestions for improving living habits and/or suggestions for going to the hospital for treatment for the user.

Using the above medical data management method provided by the present disclosure, the first client associated with the user can receive the processing scheme for the current stage of illness of the user from the server, and can supervise the user to perform treatment by himself according to the processing scheme. In addition, the first client can also output the reminding message according to the received processing scheme, so that the user can be supervised effectively.

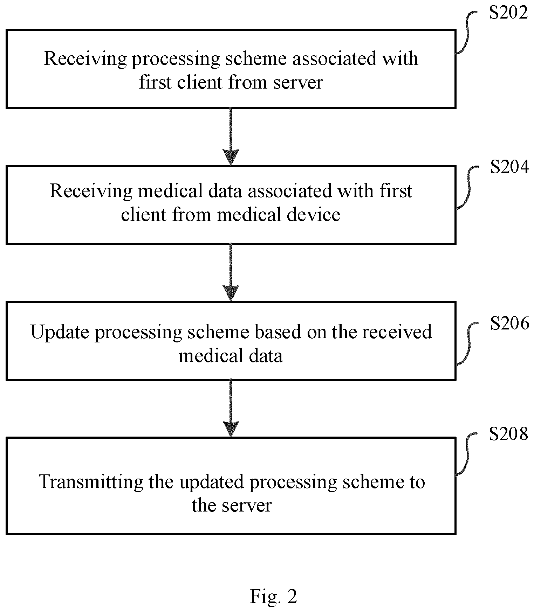

Fig. 2 illustrates another schematic flow chart of a medical data management method according to the embodiments of the present disclosure. The medical data management method illustrated in Fig. 2 may be executed by a second client. Hereinafter, the second client may refer to a client used by a doctor, which is also referred to as a doctor terminal. In some embodiments, the second client may be a client used by a doctor in a first-level hospital. The first-level hospital may be a hospital with lower level, such as a community hospital, a street hospital, a community health service station, a level-1 hospital, etc. In other embodiments, the second client may be a client used by a doctor in a second-level hospital. The second-level hospital may be a hospital with higher level, for example, the hospital rated as level 2 or above, including a level-2 hospital and level-3 hospital (such as a 3A grade hospital or a 3B grade hospital). The second-level hospitals may also include a comprehensive private hospital equipped with the required medical devices, etc.

In step S202, a processing scheme associated with the first client may be received from the server.

In the case where the user visits a hospital, the doctor can transmit a request for accessing historical record of medical data of the user to the server by using the doctor terminal, and receive a processing scheme, such as a treatment scheme, associated with the user from the server.

By obtaining the historical medical data associated with the user from the server, the doctor can conveniently know the historical medical record of the user, so as to give appropriate examination and diagnosis result.

In some embodiments, the first client and the second client may be associated with each other. For example, before step S202, the second client may output an identification associated with the doctor who uses the second client. In some examples, the identification may be formed as a bar code, a two-dimensional code or any form that can be used to identify the doctor. In this way, the first client can transmit an association request for associating with the second client according to the identification associated with the doctor. For example, the two-dimensional code of the identification associated with the doctor can be scanned by the user terminal, and then the user terminal is associated with the second client.

In other examples, the second client may also be associated with the first client according to the identification of the user of the first client. The identification of the user may include the two-dimensional code and RFID (e.g., bracelet identification) representing the identification of the user of the first client, the ID number, palm vein, and palm print of the user of the first client, and other types of identifications which can represent the identity information of the user of the first client.

In step S204, medical data associated with the first client is received from a medical device. The medical device here may include at least two of the lung function instrument, ventilator, oxygen generator, atomizer, non-invasive blood multi-parameter detector, and sleep screening instrument. Among them, the lung function instrument, sleep screening instrument, and non-invasive blood multi-parameter detector can be used to perform the required examination for the user. The ventilator, oxygen generator, and atomizer can be used to perform the required treatment for the user. In some examples, the medical device may include at least two of the above-mentioned medical devices. For example, the at least two medical devices may include at least one medical device for examination and at least one medical device for treatment.

In some embodiments, the doctor may associate the medical device to be used with the first client by using the second client. For example, the device number of the medical device can be associated with the identification of the user (such as any of the aforementioned identifications representing the identity information of the user of the first client), so that the medical data generated by the medical device is associated with the first client.

In step S204, the doctor can use the medical device to perform the required examination for the user and receive the medical data generated by the medical device. Using the received medical data, the doctor can make corresponding determinations on the user's illness based on the user's current physical condition.

In other embodiments, the medical data generated by the medical device may also be transmitted to the server, so that the server can store, in association with the first client, the medical data generated during this examination.

In step S206, the processing scheme may be updated based on the received data.

Using the medical data obtained in step S204, the doctor can update the processing scheme for the user, as a next processing scheme.

In the case where the second client is a client of the first-level hospital, the next processing scheme may include: the processing scheme for leaving hospital, the processing scheme for staying in hospital, or the processing scheme for transferring to a hospital with higher level.

For example, in the case where the received medical data indicates that the user's physical condition is good, the doctor can update the processing scheme for the user to the processing scheme for leaving hospital by using the second client. For example, the processing scheme for leaving hospital indicates that the user does not need to receive further treatment in hospital. In addition, the processing scheme for leaving hospital can also include a processing scheme for self-treatment that the user needs to follow.

For another example, in the case where the received medical data indicates that the illness of the user is in an non-stationary stage but the symptoms of the user can be controlled by a hospital with lower level, the doctor can update the processing scheme for the user to the processing scheme for staying in hospital by using the second client. For example, the processing scheme for staying in hospital indicates that the user needs to receive further treatment in the hospital with lower level. In some examples, the processing scheme for staying in hospital may also include the length of time for which the user receives treatment in the hospital with lower level and the items of treatment that the user needs to receive.

For example, in the case where the received medical data indicates that the user's illness is relatively serious, the doctor can update the processing scheme for the user to the processing scheme for transferring to the hospital with higher level. In some examples, the processing scheme for transferring to the hospital with higher level may include the name of the hospital with higher level and the designated doctor.

In some embodiments, in the case where the updated processing scheme is the processing scheme for transferring to the hospital with higher level, the doctor can transmit a transfer application to the hospital with higher level and/or doctor to be transferred to through the second client, and receive a response request transmitted by the hospital with higher level and/or doctor. In the case where the response request indicates that the hospital with higher level and/or doctor can receive the user to be transferred, the next processing scheme can be determined as the processing scheme for transferring to the hospital with higher level and/or doctor.

In the case where the second client is a client of the second-level hospital, the next processing scheme may include: the processing scheme for staying in hospital, or the processing scheme for transferring to a hospital with lower level.

For another example, in the case where the received medical data indicates that the illness of the user is in the non-stationary stage, but the symptoms of the user can be controlled by the hospital with lower level, the doctor can use the second client to update the processing scheme for the user to the processing scheme for transferring to the hospital with lower level. For example, the processing scheme for transferring to the hospital with lower level indicates that the user can receive further treatment in the hospital with lower level.

In some examples, the processing scheme for transferring to the hospital with lower level may include the name of the hospital with lower level and the designated doctor. The doctor can transmit a transfer application to the hospital with lower level and/or doctor to be transferred to through the second client, and receive the response request sent by the hospital with lower level and/or doctor. In the case where the response request indicates that the hospital with lower level and/or doctor can receive the user to be transferred, the next processing scheme can be determined as the processing scheme for transferring to the hospital with lower level and/or doctor.

In some examples, the processing scheme for transferring to the hospital with lower level and/or doctor may also include the treatment scheme for the user formulated by the doctor of the second-level hospital, such as the length of time for which the user receives treatment in the hospital with lower level and the items of treatment that the user needs to receive.

For example, in the case where the received medical data indicates that the illness of the user is relatively serious, the doctor can update the processing scheme for the user to the processing scheme for staying in hospital by using the second client. For example, the processing scheme for staying in hospital indicates that the user needs to receive further treatment in the second-level hospital. In some examples, the processing scheme for staying in hospital may also include the length of time for which the user receives treatment in the second-level hospital and the items of treatment that the user needs to receive.

In step S208, the updated processing scheme may be transmitted to the server. The updated processing scheme may be stored in the server in association with the first client. The updated processing scheme can be transmitted to the first client via the server. The updated processing scheme can also be transmitted simultaneously to the third client associated with the first client.

Using the above medical data management method provided by the present disclosure, the doctor can receive the historical medical data and medical records of the user from the server by using the client, thus the current physical condition of the user can be determined more accurately. In addition, in the case where the user needs to change the hospital for treatment, the doctor can make a referral request for the user conveniently by using the client, thus improving the communication efficiency between different hospitals.

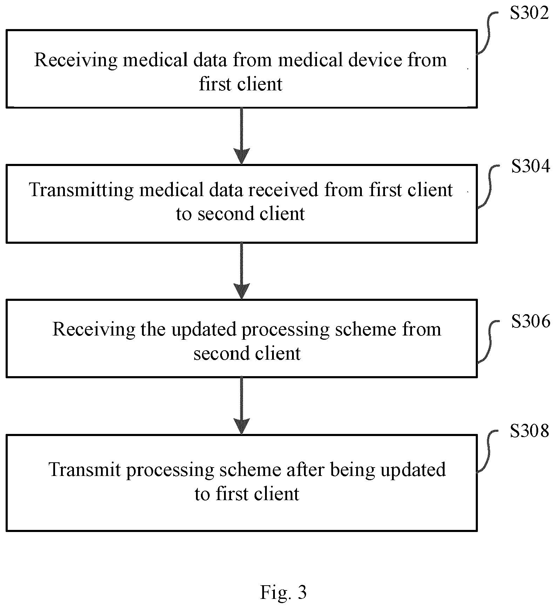

Fig. 3 illustrates another schematic flow chart of a medical data management method according to the embodiments of the present disclosure. The medical data management method illustrated in Fig. 3 may be executed by the server. The server can communicate with the first client and/or the second client through the network.

In step S302, medical data from the medical device may be received from the first client. The medical device may include the lung function instrument, ventilator, oxygen generator, atomizer, non-invasive blood multi-parameter detector, sleep screening instrument and other devices.

In an alternative embodiment, step S302 may include receiving medical data associated with the first client from the medical device. For example, in the case where the user is undergoing examination and/or treatment in the hospital, the doctor or staff member may associate the medical device with the identification of the user associated with the first client. After the examination is completed using the medical device, the server may receive the generated medical data associated with the first client from the medical device. The identification of the user may include the two-dimensional code and RFID (e.g., the bracelet identification) representing the identification of the user of the first client, the ID number, palm vein, and palm print of the user of the first client, and other identifications which can represent the identity information of the user of the first client.

In some implementations, step S302 may further include receiving the message indicating that the specified behavior has been completed from the first client. For example, the server may receive the data generated by the medical device indicating that the user has completed the specified treatment or examination behavior and/or the picture (such as a photo and/or video about the user using the medical device) and/or text message indicating that the user has completed the specified treatment or examination behavior from the first client. The server may store the received message which indicates that the specified behavior has been completed in association with the first client.

In step S304, the medical data received in step S302 may be transmitted to the second client.

In some embodiments, the first client and the second client may be associated with each other. For example, before step S304, the server may receive an association request for associating with the second client transmitted by the first client, and associate the first client with the second client in response to the received association request.

For example, the user can scan the identification (such as the two-dimensional code) corresponding to the second client by using the first client, thereby transmitting the association request for associating with the second client to the server. The server may associate the first client with the second client in response to the received association request.

In other examples, the second client may also be associated with the first client according to the identification of the user of the first client. The identification of the user may include the two-dimensional code and RFID (e.g., the bracelet identification) representing the identification of the user of the first client, the ID number, palm vein, and palm print of the user of the first client, and other identifications which can represent the identity information of the user of the first client.

Utilizing step S304, the doctor can obtain the medical data of the user through the server, including the historical examination data and treatment data of the user.

In step S306, the updated processing scheme may be received from the second client.

Utilizing the medical data obtained in step S304, the doctor can update the processing scheme for the user, as the next processing scheme. In the case where the second client is a client of the first-level hospital, the next processing scheme may include: the processing scheme for leaving hospital, the processing scheme for staying in hospital, or the processing scheme for transferring to the hospital with higher level.

In step S308, the updated processing scheme may be transmitted to the first client.

In this way, the user of the first client can conveniently obtain the next processing scheme determined by the doctor. For example, in the case where the updated processing scheme indicates that the user can leave the hospital, the user can obtain the processing scheme for self-treatment that the user needs to follow when leaving the hospital for treatment through the server. In the case where the updated processing scheme indicates that the user needs to stay in hospital for treatment, the user can obtain the length of time for which the user receives treatment in the hospital and the items of treatment that the user needs to receive through the server. In the case where the updated processing scheme indicates that the user needs to be transferred to another hospital (for example, a hospital with higher level or lower level) for treatment, the user can obtain the name of the hospital to be transferred to and the designated doctor through the server.

Utilizing the medical data management method provided by the present disclosure, the server can share the medical data associated with the user with the doctors at respective levels, and can synchronize the processing results of the doctors to the user. Therefore, the efficiency of communication and information acquisition between the doctors and the user can be improved in the visit process.

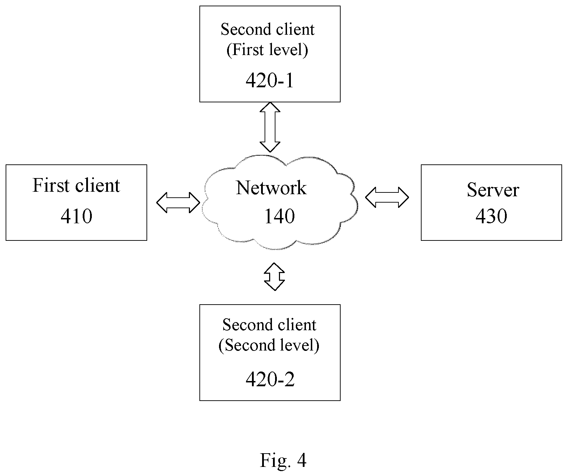

Fig. 4 illustrates a schematic diagram of a medical data management system according to the embodiments of the present disclosure. As illustrated in Fig. 4, the medical data management system may include a first client 410, a second client 420, and a server 430. The first client may refer to the client used by the user, which is also referred to as the user terminal. The second client may refer to the client used by the doctor, which is also referred to as the doctor terminal. In some embodiments, the second client may be the client 420-1 used by the doctor in the first-level hospital. The first-level hospital may be the hospital with lower level, such as a community hospital, a street hospital, a community health service station, a level-1 hospital, etc. In other embodiments, the second client may be the client 420-2 used by the doctor in the second-level hospital. The second-level hospital can be the hospital with higher level, for example, the hospital rated as level 2 or above, including a level-2 hospital, a level-3 (such as the 3A grade hospital or the 3B grade hospital). The second-level hospital can also include the comprehensive private hospital equipped with the required medical devices, etc.

It can be understood that classifying the hospitals into the first-level hospital with the lower level and the second-level hospital with the higher level has been illustrated as only one example above. The hospitals can be classified in any manner without departing from the principles of the present disclosure.

In some embodiments, the first client 410 and the second client 420 may be implemented as any other type of electronic devices capable of performing data processing, which may include but not limited to desktop computers, notebook computers, tablet computers, smart phones, smart home devices, wearable devices, vehicle-mounted electronic devices, monitoring devices, medical electronic devices, and the like.

The server 430 may be a single server or a group of servers, and each server in the group are connected through wired or wireless networks. The group of servers can be centralized, such as a data center, or distributed. The server 430 may be local or remote.

In some embodiments, the first client 410, the second client 420, and the server 430 can communicate through the network 140. The network may be a single network or a combination of at least two different networks. For example, the network may include, but is not limited to, one of or a combination of several of local area network, wide area network, public network, private network, etc.

In some embodiments, the first client 410, the second client 420, and the server 430 may be connected to a database. The database can generally refer to a device or unit having a storage function. The database is mainly used for storing various data utilized, generated and output by the first client 410, the second client 420, and the server 430 during working. The database can be local or remote. The database may include various memories, such as random access memories (RAMs), read only memories (ROMs), and the like. The above-mentioned storage devices are just some examples, and the storage devices used in the system are not limited to this.

In some embodiments, the medical data processing system 400 may further include a third client (not illustrated). For example, the third client may be a client associated with the first client. In some examples, the third client may be a client used by a relative/friend of the user. The server can synchronize the message transmitted to the first client (e.g., the reminding message or processing scheme) to the third client.

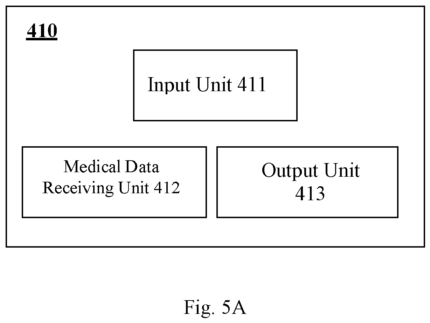

Fig. 5A illustrates a schematic block diagram of the first client according to the embodiments of the present disclosure. The first client illustrated in Fig. 5A can be configured to execute the method flow illustrated in Fig. 1. In some embodiments, the method flow illustrated in Fig. 1 may be executed using the application programs built in the first client.

As illustrated in Fig. 5A, the first client 410 may include an input unit 411, a medical data receiving unit 412, and an output unit 413.

The input unit 411 may be configured to receive a processing scheme associated with the first client from a server. In some embodiments, the first client receives a user input and transmits the request for acquiring an associated processing scheme to the server in response to the user input, thereby receiving the associated processing scheme. In other embodiments, the first client may transmit the request for acquiring the associated processing scheme to the server at a fixed period (e.g., every day, every week, every month, or any other predefined time period), so as to receive the associated processing scheme. In yet other embodiments, the first client may receive the processing scheme actively transmitted by the server.

In the case where the user uses the first client, the user can log in through a predefined user identification, so that the first client is associated with the user identification.

In some embodiments, the processing scheme may be a treatment scheme of the user corresponding to the first client.

In some implementations, the treatment scheme may be the medication scheme that indicates the user to take his own medicines at home. For example, the medication scheme may include the user using a medical device for the required treatment and/or examination at a predefined time. For example, the medical device may include a lung function instrument, a ventilator, an oxygen generator, an atomizer, a non-invasive blood multi-parameter detector, and a sleep screening instrument. Among them, the lung function instrument, the sleep screening instrument, and the non-invasive blood multi-parameter detector can be used to perform the required examination for the user. The ventilator, the oxygen generator, and the atomizer can be used to perform the required treatment for the user. In some examples, the medical device may include at least two of the above-mentioned medical devices. For example, the at least two medical devices may include at least one medical device for examination and at least one medical device for treatment.

In other implementations, the treatment scheme may indicate that the user should go to a hospital for treatment. For example, the treatment scheme may include the user going to a designated hospital regularly for reexamination. For another example, the treatment scheme may include the user going to a hospital for examination when the medical data of the user is abnormal.

The medical data receiving unit 412 may be configured to receive medical data corresponding to the processing scheme from a medical device associated with the first client.

In some embodiments, the device such as the lung function instrument, ventilator, oxygen generator, atomizer, non-invasive blood multi-parameter detector, and sleep screening instrument can be associated with the user terminal, and the usage data of the device can be transmitted to the user terminal. For example, in the case where the user uses the medical device, the user terminal can receive the medical data generated by the medical device. In some examples, the user terminal may receive the medical data from the medical device through the wireless network such as Bluetooth or Wifi or any wired network.

In some embodiments, the first client 410 may further include a reminding unit. The reminding unit may be configured to determine that the user should receive treatment through a specific medical device (e.g., the atomizer) at a predefined time according to the received treatment scheme. In some examples, the reminding unit may determine the reminding message for reminding the user to use the atomizer for treatment at a period of time before the predefined time (e.g., five minutes, ten minutes or other predefined time intervals before the predefined time), and output the reminding message. In other examples, if the user terminal does not receive the medical data from the atomizer at the predefined time, the reminding unit may determine the reminding message for reminding the user to use the atomizer for treatment and then output the reminding message. For example, if the user has not completed the specified treatment or examination behavior when a period of time (for example, one hour, two hours or other predefined time intervals) after the specified treatment time ends, the reminding unit can output the reminding message above.

In some embodiments, the first client may transmit the message indicating that the specified behavior has been completed to the server so as to cancel the reminder. For example, the first client may transmit the received data from the medical device indicating that the user has completed the specified treatment or examination behavior to the server so as to indicate that the specified behavior has been completed. For another example, the first client may also transmit the picture (e.g., the photo and/or video about the user using the medical device) and/or text message indicating that the user has completed the specified treatment or examination behavior to the server, so as to indicate that the specified behavior has been completed.

In still other examples, the first client 410 may further include a forwarding unit. The forwarding unit may forward the reminding message to the third client associated with the first client. For example, the third client may be the client used by a relative/friend of the user associated with the first client. The forwarding unit may forward the reminding message to the third client via the server.

The output unit 413 may be configured to transmit the received medical data to the server. For example, the user terminal can transmit the received medical data to the server via any wireless network and wired network. The server can record the received medical data in the medical file associated with the user corresponding to the user terminal. The doctor can directly call the history record of these pieces of medical data from the server through the client in the subsequent visit and treatment process of the user.

In some embodiments, the user can also use the first client to communicate with the doctor. For example, the user can transmit the description of his recent physical condition to the doctor through the first client. The doctor can view the user's description and give an answer, analysis, and visit reminder for the user's description through the doctor terminal. In this case, the user can receive the answer, analysis, and visit reminder given by the doctor through the first client. For example, the visit reminder may include the hospital recommended by the doctor.

In some embodiments, the first client may provide an evaluation scheme to the user. For example, the user can fill in an assessment questionnaire for the quality of life of the COPD user (CAT questionnaire) provided by the first client before going to the hospital for treatment. The first client can transmit the questionnaire filling results of the user to the client used by the doctor, and receive the assessment results made by the doctor according to the questionnaire filling results. The assessment results may include suggestions for improving living habits and/or suggestions for going to the hospital for treatment for the user.

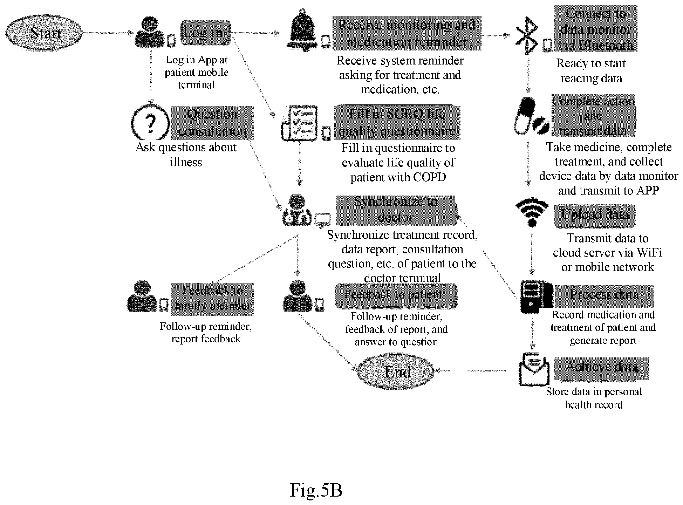

Fig. 5B illustrates a schematic diagram of an exemplary usage process of a first client in a home scene.

In the home scene, the user can log in to an application of the user's mobile terminal by using the first client. If it is the first time for the user to use the medical data management system, he is required to provide the default information required by the system (such as identity information, health condition, etc.). In this case, the normal use interface is displayed with the guidance of the system boot interface. If it is not the first time for the user to use this system, then the normal use interface of the system is directly displayed.

The application program of the first client may include three main functions: a reminding function, a consulting function, and an evaluation function.

The first client can receive the reminder from the system about monitoring behaviors such as treatment and medication. The user can active the network of the first user terminal (e.g., Bluetooth switch) and connect the associated (e.g., previously accessed) medical device (the ventilator or atomizer, etc.). After the treatment, the data generated by the medical device can be transmitted to the first client via Bluetooth or Wifi, and the first client can transmit the data to the cloud server via WiFi or mobile network. The cloud server can generate a report according to the user's medication and treatment conditions, and record the data in a personal health record, and the health record of the user may eventually be synchronized to the doctor terminal.

In order to improve the compliance of the user, the system also provides the function of binding a family member, and the reminder about monitoring behaviors such as treatment and medication can be transmitted to the family member simultaneously. If the user has not completed the specified behavior when two hours after the treatment time end, a message may be pushed to the family member, so that the family member can remind the user to take treatment or take medicine.

The user can use the first client to consult the doctor about his own recent physical condition or illness. According to the user's description, the doctor may give an answer to the question, an analysis for the medical report, and a follow-up reminder in combination with the user's health record and the examination result uploaded to the system, and may feed the relevant result back to the user's family member simultaneously. When the hospital for the user to recheck is to be determined, corresponding filtering conditions can be set in the system. The filtering conditions may include the distance between the hospital and the user, the specialty ranking of the corresponding department of the hospital, the difficulty of hospital registration, etc. The system can recommend the most appropriate hospital according to the filtering conditions selected by the user.

In addition, the user can use the first client for health assessment. Taking the chronic obstructive pulmonary disease as an example, the user can fill in the CAT questionnaire or SGRQ questionnaire. The doctor can assess the life quality of the user through the score of the questionnaire, and give corresponding suggestions such as improving living habits or going to the hospital for treatment according to the assessment result.

Using the above medical data management apparatus provided by the present disclosure, the first client associated with the user can receive the processing scheme for the current stage of illness of the user from the server, and can supervise the user to perform treatment by himself according to the processing scheme. In addition, the first client can also output the reminding message according to the received processing scheme, so that the user can be supervised effectively.

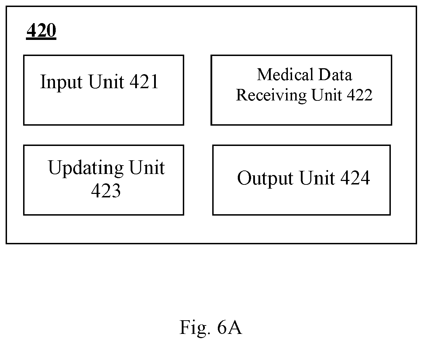

Fig. 6A illustrates a schematic block diagram of the second client according to the embodiments of the present disclosure. The second client illustrated in Fig. 6A may be configured to execute the method flow illustrated in Fig. 2. In some embodiments, the method flow illustrated in Fig. 2 may be performed using the application built in the second client. The second client may refer to the client used by the doctor, which is also referred to as the doctor terminal. In some embodiments, the second client may be the client used by the doctor in the first-level hospital. The first-level hospital may be the hospital with lower level, such as a community hospital, a street hospital, a community health service station, a level-1 hospital, etc. In other embodiments, the second client may be the client used by the doctor in the second-level hospital. The second-level hospital can be the hospital with higher level, for example, the hospital rated as level 2 or above, including a level-2 hospital, a level-3 (such as the 3A grade hospital or the 3B grade hospital). The second-level hospital can also include the comprehensive private hospital equipped with the required medical devices, etc.

As illustrated in Fig. 6A, the second client 420 may include an input unit 421, a medical data receiving unit 422, an updating unit 423, and an output unit 424.

The input unit 421 may be configured to receive a processing scheme associated with a first client from a server.

In the case where the user visits a hospital, the doctor can transmit a request for accessing historical records of medical data of the user to the server by using the doctor terminal, and receive a processing scheme, such as a treatment scheme, associated with the user from the server.

By obtaining the historical medical data associated with the user from the server, the doctor can conveniently know the historical medical record of the user, so as to give appropriate examination and diagnosis result.

In some embodiments, the first client and the second client may be associated with each other. For example, the second client may output the identification associated with the doctor who uses the second client. In some examples, the identification may be formed as a bar code, a two-dimensional code or any form that can be used to identify the doctor. In this way, the first client can transmit a request for associating with the second client according to the identification associated with the doctor. For example, the two-dimensional code of the identification associated with the doctor can be scanned by the user terminal, and then the user terminal is associated with the second client.

In other examples, the second client may also be associated with the first client according to the identification of the user of the first client. The identification of the user may include the two-dimensional code and RFID (e.g., bracelet identification) representing the identification of the user of the first client, the ID number, palm vein, and palm print of the user of the first client, and other identifications which can represent the identity information of the user of the first client.

The medical data receiving unit 422 may be configured to receive the medical data associated with the first client from a medical device. The medical device here may include the lung function instrument, ventilator, oxygen generator, atomizer, non-invasive blood multi-parameter detector, and sleep screening instrument. Among them, the lung function instrument, sleep screening instrument, and non-invasive blood multi-parameter detector can be used to perform the required examination for the user. The ventilator, oxygen generator, and atomizer can be used to perform the required treatment for the user. In some examples, the medical device may include at least two of the above-mentioned medical devices. For example, the at least two medical devices may include at least one medical device for examination and at least one medical device for treatment.

In some embodiments, the doctor may associate the medical device to be used with the first client by using the second client. For example, the device number of the medical device can be associated with the identification of the user (such as any of the aforementioned identifications representing the identity information of the user of the first client), so that the medical data generated by the medical device is associated with the first client.

The doctor can use the medical device to perform the required examination for the user and receive the medical data generated by the medical device. Using the received medical data, the doctor can make corresponding determinations on the user's illness based on the user's current physical condition.

In other embodiments, the medical data generated by the medical device may also be transmitted to the server, so that the server can store, in association with the first client, the medical data generated during this examination.

The updating unit 423 may be configured to update the processing scheme based on the received data.

Using the medical data acquired by the medical data receiving unit 422, the doctor can update the processing scheme for the user, as the next processing scheme.

In the case where the second client is a client of the first-level hospital, the next processing scheme may include: the processing scheme for leaving hospital, the processing scheme for staying in hospital, or the processing scheme for transferring to a hospital with higher level.

For example, in the case where the received medical data indicates that the user's physical condition is good, the doctor can update the processing scheme for the user to the processing scheme for leaving hospital by using the second client. For example, the processing scheme for leaving hospital indicates that the user does not need to receive further treatment in hospital. In addition, the processing scheme for leaving hospital can also include a processing scheme for self-treatment that the user needs to follow.

For another example, in the case where the received medical data indicates that the illness of the user is in an non-stationary stage but the symptoms of the user can be controlled by a hospital with lower level, the doctor can update the processing scheme for the user to the processing scheme for staying in hospital by using the second client. For example, the processing scheme for staying in hospital indicates that the user needs to receive further treatment in the hospital with lower level. In some examples, the processing scheme for staying in hospital may also include the length of time for which the user receives treatment in the hospital with lower level and the items of treatment that the user needs to receive.

For example, in the case where the received medical data indicates that the user's illness is relatively serious, the doctor can update the processing scheme for the user to the processing scheme for transferring to the hospital with higher level. In some examples, the processing scheme for transferring to the hospital with higher level may include the name of the hospital with higher level and the designated doctor.

In some embodiments, in the case where the updated processing scheme is the processing scheme for transferring to the hospital with higher level, the doctor can transmit a transfer application to the hospital with higher level and/or doctor to be transferred to through the second client, and receive a response request transmitted by the hospital with higher level and/or doctor. In the case where the response request indicates that the hospital with higher level and/or doctor can receive the user to be transferred, the next processing scheme can be determined as the processing scheme for transferring to the hospital with higher level and/or doctor.

In the case where the second client is a client of the second-level hospital, the next processing scheme may include: the processing scheme for staying in hospital, or the processing scheme for transferring to a hospital with lower level.

For another example, in the case where the received medical data indicates that the illness of the user is in the non-stationary stage, but the symptoms of the user can be controlled by the hospital with lower level, the doctor can use the second client to update the processing scheme for the user to the processing scheme for transferring to the hospital with lower level. For example, the processing scheme for transferring to the hospital with lower level indicates that the user can receive further treatment in the hospital with lower level.

In some examples, the processing scheme for transferring to the hospital with lower level may include the name of the hospital with lower level and the designated doctor. The doctor can transmit a transfer application to the hospital with lower level and/or doctor to be transferred to through the second client, and receive the response request sent by the hospital with lower level and/or doctor. In the case where the response request indicates that the hospital with lower level and/or doctor can receive the user to be transferred, the next processing scheme can be determined as the processing scheme for transferring to the hospital with lower level and/or doctor.

In some examples, the processing scheme for transferring to the hospital with lower level and/or doctor may also include the treatment scheme for the user formulated by the doctor of the second-level hospital, such as the length of time for which the user receives treatment in the hospital with lower level and the items of treatment that the user needs to receive.

For example, in the case where the received medical data indicates that the illness of the user is relatively serious, the doctor can update the processing scheme for the user to the processing scheme for staying in hospital by using the second client. For example, the processing scheme for staying in hospital indicates that the user needs to receive further treatment in the second-level hospital. In some examples, the processing scheme for staying in hospital may also include the length of time for which the user receives treatment in the second-level hospital and the items of treatment that the user needs to receive.

The output unit 424 may be configured to transmit the updated processing scheme to the server. The updated processing scheme may be stored in the server in association with the first client. The updated processing scheme can be transmitted to the first client via the server. The updated processing scheme can also be transmitted simultaneously to the third client associated with the first client.

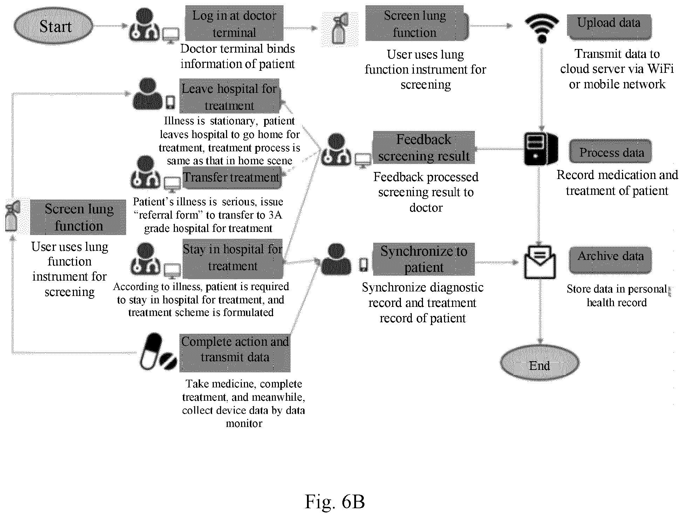

Fig. 6B illustrates a schematic diagram of an exemplary usage process of the second client in the first-level hospital scene.

In the first-level hospital scene, the doctor can log in to the system using the second client. When the user visits the doctor, the user can scan the two-dimensional code of the doctor so that the doctor and the user can be bound to each other.

During the examination, the doctor binds the medical device, such as the lung function instrument, to the user (for example, matches the device number of the lung function instrument with the mobile phone number of the designated user), and lung function of the user is then screened. The medical data generated by the medical device can be transmitted to the cloud server via WiFi or mobile network. If it is the first time for the user to visit a doctor, the server may record the lung function examination result of the user, record the data in the user's personal health record, and push it to the previously bound doctor. If it is not the first time for the user to visit a doctor, the server may compare the lung function examination result with the previous lung function examination results of the user, call the user's medication and treatment conditions, feed the comparison result and treatment record back to the bound doctor, and record the newly generated medical data in the user's personal health record.

The doctor can determine a treatment mode to be applied to the user according to the comparison result of the received historical medical data of the user and the medical data obtained from this examination, in which: (1) if the user's illness is stationary, he can leave the hospital and go home for treatment; (2) if the user's illness is in the non-stationary stage but the community hospital can handle it, he needs to stay in hospital for treatment, the doctor can formulate the treatment scheme according to the physical condition of the user, after the treatment is completed, the diagnosis record and treatment record of the user are synchronized to the client, after treatment, the doctor can examine the lung function of the user again, and determine whether it meets the standard of leaving hospital for treatment; (3) if the user's illness is serious, the doctor can transfer the user to the hospital with higher level (e.g., the level 3A grade hospital in the second-level hospitals) for treatment, the doctor can issue a "referral form" and transmit a referral request to the server, so that the user can be transferred to the hospital with higher level for treatment, and after the user completes the treatment, the system can synchronize the diagnosis record and treatment record of the user in the hospital with higher level to the user terminal.

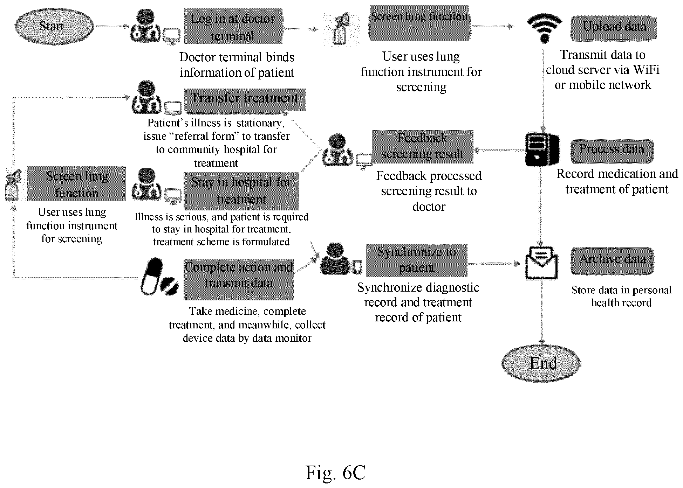

Fig. 6C illustrates a schematic diagram of an exemplary usage process of the second client in the second-level hospital scene.

In the second-level hospital scene, the doctor can log in to the system using the second client. When the user visits the doctor, the user can scan the two-dimensional code of the doctor so that the doctor and the user can be bound to each other.

During the examination, the doctor binds the medical device, such as the lung function instrument, to the user (for example, matches the device number of the lung function instrument with the mobile phone number of the designated user), and lung function of the user is then screened. The medical data generated by the medical device can be transmitted to the cloud server via WiFi or mobile network. If it is the first time for the user to see the doctor, the server may record the lung function examination result of the user, record the data in the user's personal health record, and push it to the previously bound doctor. If it is not the first time for the user to see the doctor, the server may compare the lung function examination result with the previous lung function examination results of the user, call the user's medication and treatment conditions, feed the comparison result and treatment record back to the bound doctor, and record the newly generated medical data in the user's personal health record.

The doctor can determine the treatment mode to be applied to the user according to the comparison result of the received historical medical data of the user and the medical data obtained from this examination, in which: (1) if the user's illness is in the non-stationary stage but the hospital with lower level (for example, the community hospital in the first-level hospitals) can handle it, the doctor can issue the "referral form" and transmit the referral request to the server, so that the user can be transferred to the hospital with lower level for treatment, and after the treatment is completed, the diagnosis record and treatment record of the user in the hospital with higher level are synchronized to the user terminal; (2) if the user's illness is serious, he needs to stay in hospital for treatment, the doctor can make the treatment scheme according to the physical condition of the user, after the treatment is completed, the diagnosis record and treatment record of the user are synchronized to the user terminal, and after treatment, the doctor can examine the lung function of the user again, and determine whether it meets the standard of leaving hospital for treatment.

Using the above medical data management apparatus provided by the present disclosure, the doctor can receive the historical medical data and medical record of the user from the server by using the client, thus the current physical condition of the user can be determined more accurately. In addition, in the case where the user needs to change the hospital, the doctor can make a referral request for the user conveniently by using the client, thus improving the communication efficiency between different hospitals.

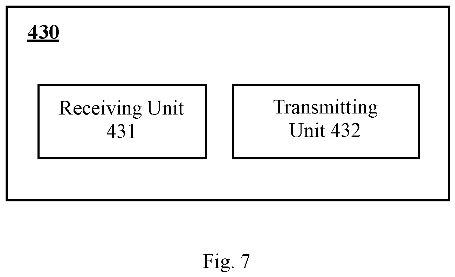

Fig. 7 illustrates a schematic block diagram of the server according to the embodiments of the present disclosure. The server illustrated in Fig. 7 may be configured to execute the method flow illustrated in Fig. 3. In some embodiments, the method flow illustrated in Fig. 3 may be performed using the application built in the server.

As illustrated in Fig. 7, the server 430 may include a receiving unit 431 and a transmitting unit 432.

The receiving unit 431 may be configured to receive medical data from a medical device from a first client. For example, the medical device may include the lung function instrument, ventilator, oxygen generator, atomizer, non-invasive blood multi-parameter detector, and sleep screening instrument. Among them, the lung function instrument, the sleep screening instrument, and the non-invasive blood multi-parameter detector can be used to perform the required examination for the user. The ventilator, the oxygen generator, and the atomizer can be used to perform the required treatment for the user. In some examples, the medical device may include at least two of the above-mentioned medical devices. For example, the at least two medical devices may include at least one medical device for examination and at least one medical device for treatment.

In an alternative embodiment, the receiving unit 431 may also be configured to receive a message from the first client indicating that the specified behavior has been completed. For example, in the case where the user is undergoing examination in the hospital, the doctor or staff member may associate the medical device with the identification of the user associated with the first client. After the examination is completed using the medical device, the server may receive the generated medical data associated with the first client from the medical device.

In some implementations, the receiving unit 431 may be further configured to receive the message indicating that the specified behavior has been completed from the first client. For example, the server may receive the data generated by the medical device indicating that the user has completed the specified treatment or examination behavior and/or the picture (such as a photo and/or video about the user using the medical device) and/or text message indicating that the user has completed the specified treatment or examination behavior from the first client. The server may store the received message which indicates that the specified behavior has been completed in association with the first client.

The transmitting unit 432 may be configured to transmit the medical received data by the receiving unit 431 to the second client.

In some embodiments, the first client and the second client may be associated with each other. For example, the server may further include an association unit, and the association unit may be configured to receive the association request for associating with the second client transmitted by the first client, and associate the first client with the second client in response to the received association request.

For example, the user can scan the identification (such as the two-dimensional code) corresponding to the second client by using the first client, thereby transmitting the association request for associating with the second client to the server. The server may associate the first client with the second client in response to the received association request.

Further, the receiving unit 431 may be configured to receive the updated processing scheme from the second client.

Using the medical data of the user received from the server, the doctor can update the processing scheme for the user as the next processing scheme. In the case where the second client is a client of the first-level hospital, the next processing scheme may include:
the processing scheme for leaving hospital, the processing scheme for staying in hospital, or
the processing scheme for transferring to the hospital with higher level.

Further, the transmitting unit 432 may be configured to transmit the updated processing scheme to the first client.

In this way, the user of the first client can conveniently obtain the next processing scheme determined by the doctor. For example, in the case where the updated processing scheme indicates that the user can leave the hospital, the user can obtain the processing scheme for self-treatment that the user needs to follow when leaving the hospital for treatment through the server. In the case where the updated processing scheme indicates that the user needs to stay in hospital for treatment, the user can obtain the length of time for which the user receives treatment in the hospital and the items of treatment that the user needs to receive through the server. In the case where the updated processing scheme indicates that the user needs to be transferred to other hospitals (for example, the hospital with higher level or lower level) for treatment, the user can obtain the name of the hospital to be transferred to and the designated doctor through the server.

Utilizing the medical data management server provided by the present disclosure, the server can share the medical data associated with the user with the doctors at respective levels, and can synchronize the processing results of the doctors to the user. Therefore, the efficiency of communication and information acquisition between the doctors and the user can be improved in the visit process.

According to another embodiment of the present disclosure, there is also provided a medical data management method 800, which is applied to the user terminal and includes the following steps.

In step 802, the user terminal binds its corresponding user with a doctor.

For example, the user can scan the two-dimensional code of the doctor (e.g., via WeChat) by using the user terminal, so as to input the personal information of the user for registration. After successful registration, the user is bound to the doctor for establishing the doctor-patient binding relationship.

In step 804, the user terminal transmits information of the user to a server.

For example, the user terminal can transmit the personal information input by the user when registering and the answer information of the user with respect to at least one screening questionnaire to the server.

For example, the options of the screening questionnaires are displayed on the user interface after the user has successfully registered, and the user terminal receives the user's selection (for example, click) for a screening questionnaire and displays the screening questionnaire. The user terminal then receives the input of the user to generate the answer information with respect to the screening questionnaire.

In step 806, the user terminal obtains treatment scheme for the user given by a doctor from a server, the treatment scheme includes using at least one medical device for examination, and medical data generated by the at least one medical device is processed and recorded by the server.

For example, the doctor can also obtain the information of the user from the server, so as to determine the treatment scheme for the user based on the information of the user, for example, the treatment scheme of using the lung function instrument to screen the lung function.

At least one medical device (such as the lung function instrument) uploads the generated medical data to the server, the server may process and record the medical data, generate the medical report, and associate the medical data with the user's personal health record, for being obtained by the client such as the doctor terminal. Alternatively, the lung function instrument can also communicate with other output devices (such as the POS printer), for example, through Bluetooth connection, so that the medical data can be stored at the other output devices and can be printed at any time.

In step 808, the user terminal acquires the medical data associated with the user generated by the at least one medical device from the server and displays the medical data.

For example, in order for the user to view the medical data in real time, the user terminal transmits an acquisition request to the server, and the acquisition request may optionally include the identification information of the user, so that the server returns the medical data or the corresponding medical report to the user terminal for display on the user interface of the user terminal.

In some embodiments, the medical data management method 800 may further include step S810, in which the user terminal receives a referral notification from the server.

For example, in the case where the doctor thinks that the user is not suitable for treatment in the present hospital (for example, if the patient's illness is serious, he needs to be transferred to the hospital with higher level, or if the patient's illness is alleviated, he can be transferred to the hospital with lower level), the doctor can transmit a referral request to another doctor in other hospitals, and the server can transmit the referral notification to the user terminal after the other doctor accepts the referral request.

In addition, if the treatment scheme includes staying at home for treatment, the doctor can provide another screening questionnaire (for example, the CAT questionnaire) to the user terminal, the screening questionnaire being transmitted through the server, so that the user terminal presents the screening questionnaire and/or the interface for the user to fill in the medication record, and receives the user's answer information, for transmitting it to the server, and the server can process and record the answer information. The doctor can obtain the answer information from the server, and know the condition of the user when he is staying at home for treatment according to the answer information.

Using the above-mentioned medical data management method provided by the present disclosure, the user terminal associated with the user can receive the treatment scheme for the current stage of illness of the user from the server, and can obtain the relevant data from the medical device according to the treatment scheme.

According to another embodiment of the present disclosure, there is also provided a medical data management method 900, which is applied to the doctor terminal and includes the following steps.

In step S902, the doctor terminal obtains information of a bounded user from a server and displays the information of the user.

For example, the information of the user may include personal information of the user as described above, and the answer information of the user with respect to at least one screening questionnaire.

In step S904, the doctor terminal receives a first input of a doctor to generate a treatment scheme for the user and transmits the treatment scheme to the server. The treatment scheme includes using at least one medical device for examination.

For example, when the doctor determines the treatment scheme, he needs to select one or more option from various options or input information in the text box on the user interface of the doctor terminal, so that the information corresponding to the treatment scheme is generated for being transmitted to the server.

In step S906, the doctor terminal acquires medical data associated with the user generated by the at least one medical device from the server and displays the medical data.

For example, the doctor terminal can transmit an acquisition request to the server after receiving the input of the user (for example, clicking the "view" button), and the acquisition request can optionally include the identification information of the user, so that the server returns the medical data or the corresponding medical report to the doctor terminal, for displaying on the user interface of the doctor terminal.

In step S908, the doctor terminal receives the second input from the doctor to indicate the subsequent treatment mode for the user determined based on the acquired medical data and transmits the treatment mode to the server. In the case where the doctor terminal is the client of the first-level hospital, the treatment mode includes leaving hospital for treatment, staying in hospital for treatment, or transferring to the second-level hospital higher than the first-level hospital for treatment, and in the case where the doctor terminal is the client of the second-level hospital, the treatment mode includes the staying in hospital for treatment, or transferring to the first-level hospital for treatment.

For example, after obtaining the medical data or the corresponding medical report, the doctor can determine the subsequent treatment mode for the user. In this case, the doctor inputs to the doctor terminal (for example, through the selection of the option "leaving hospital for treatment," "staying in hospital for treatment," or "transferring to another hospital for treatment") to generate the information for the subsequent treatment mode and transmit the information to the server.

In some embodiments, the medical data management method 900 may further include, for example, before step S902, the doctor terminal obtains the identification information of the doctor terminal from the server and displays the identification information, for the doctor terminal to bind with the user.

For example, the user can scan the identification information (such as the two-dimensional code) of the doctor (e.g., via WeChat) by using the user terminal, so as to input the personal information of the user for registration. After successful registration, the user is bound to the doctor for establishing the doctor-patient binding relationship.

In other implementations, the method may further include steps S910-S912 in the case where the treatment mode is related to transferring to another hospital for treatment (for example, transfer to the hospital with higher level or lower level). In step S910, the doctor terminal receives the referral related information input by the doctor and transmits the referral related information to the server, so that the server transmits the referral request to another doctor terminal. In step S912, the doctor terminal receives the referral response from another doctor terminal to the server. For example, the server transmits the referral notification to the user terminal of the user based on the referral response.

For example, in the case where the doctor thinks that the user is not suitable for treatment in the present hospital (for example, the patient whose illness is serious needs to be transferred to the hospital with higher level or the patient whose illness is alleviated can be transferred to the hospital with lower level), the doctor can input the referral related information to the corresponding doctor terminal, and transmit the referral request to another doctor terminal corresponding to another doctor in other hospitals via the server. After another doctor accepts the referral request, another doctor terminal corresponding to another doctor can transmit the referral response to the server, so that the server can transmit the referral notification to the user terminal.

In addition, the treatment scheme may also include staying at home for treatment for the user. In this case, the doctor terminal can receive the answer information of the user for another screening form, such as the CAT answer sheet and medication record, from the server to monitor the staying at home for treatment situation of the user.

Using the above medical data management method provided by the present disclosure, the doctor terminal can receive the information of the user from the server, determine the treatment scheme based on the information of the user, and obtain the relevant data from the medical device. In addition, the information can be communicated between different doctor terminals through the server to complete the referral.

According to another embodiment of the present disclosure, there is also provided a medical data management method 1000, which is applied to the server and includes the following steps.

In step S1002, the server acquires the information of a bound user from a user terminal and transmits the information of the user to a doctor terminal.

In step S1004, the server receives a treatment scheme of a doctor for the user from the doctor terminal and transmits the treatment scheme to the user terminal, and the treatment scheme includes using at least one medical device for examination.

In step S1006, the server acquires medical data associated with the user from the at least one medical device, processes and records the medical data, and transmits the medical data to the user terminal and the doctor terminal.

In step S1008, the server receives information indicating the subsequent treatment mode for the user determined based on the acquired medical data from the doctor terminal.

In other implementations, the medical data management method 1000 may further include the following steps S1010-S1014 in the case where the treatment mode is related to transferring to another hospital treatment (for example, transferring to a hospital with higher level or lower level).

In step S1010, the server receives referral related information from the doctor terminal.

In step S1012, the server transmits a referral request to another doctor terminal based on the referral related information.

In step S1014, the server transmits a referral notification to the user terminal in response to receiving a referral response from another doctor terminal.

More details of the above-mentioned operations of the server are similar to those of the user terminal and the doctor terminal, which may not be repeated here.

Using the medical data management method applied to the server provided by the embodiments of the present disclosure, the server can enable the medical data associated with the user to be shared among the doctors at respective levels, can synchronize the processing results of the doctors to the user, and can implement the referrals between the doctors. Therefore, the efficiency of communication and information acquisition between the doctors and the user in the visit process can be improved.

In addition, the method or apparatus according to the embodiments of the present disclosure can also be implemented by means of the architecture of a computing device. The computing device includes a memory and a processor, and various data or files and computer executable instructions used for processing and/or communication are stored on the memory. When the computer executable instructions are executed, the processor is caused to execute the medical data management method according to the embodiments of the present disclosure.

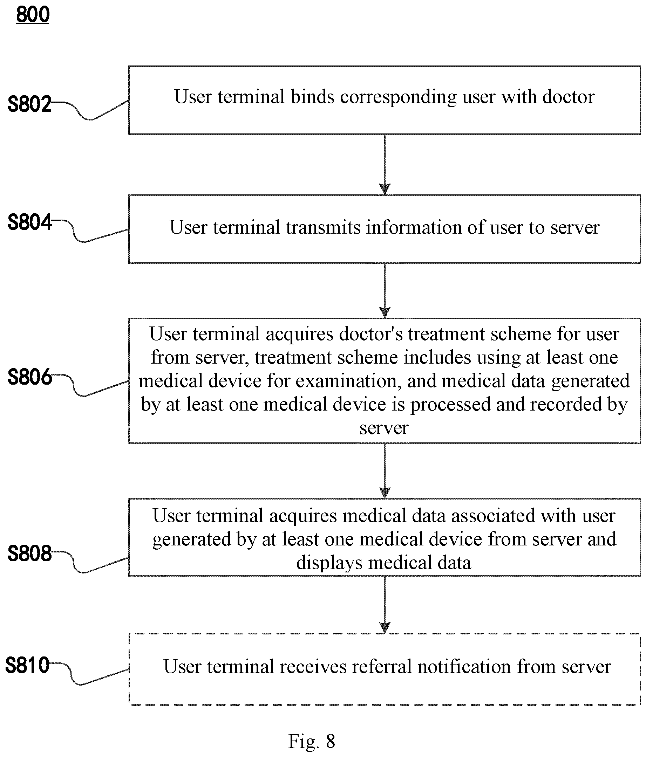

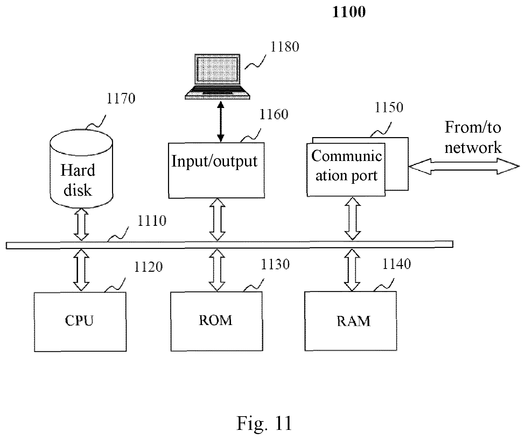

Fig. 11 illustrates an architecture of a computing device. As illustrated in Fig. 11, the computing device 1100 may include a bus 1110, one or at least two CPU 1120, a read only memory (ROM)1130, a random access memory (RAM)1140, a communication port 1150 connected to a network, an input/output component 1160, a hard disk 1170, and the like. A storage device in the computing device 1100, such as the ROM 1130 or the hard disk 1170, can store various data or files and program instructions executed by the CPU used for processing and/or communication of the medical data management method provided by the present disclosure. The computing device 1100 may also include a user interface 1180. Of course, the architecture illustrated in Fig. 11 is only exemplary, and when being implemented as a different device, one or at least two components of the computing device illustrated in Fig. 8 can be omitted according to actual needs.

According to another aspect of the present disclosure, there is also provided a non-volatile computer-readable storage medium on which computer-readable instructions are stored, and when the instructions are executed by the computer, the method as described above can be performed.

The program part in the technology can be regarded as "product" or "article" in the form of executable code and/or related data, which is implemented by the computer-readable medium. The tangible and permanent storage medium can include the memory or storage used by any computer, processor, or similar device or related module. For example, various semiconductor memories, tape drives, disk drives, or any similar devices that can provide the storage functions for software.

All or part of the software may sometimes communicate through the network, such as the Internet or other communication networks. Such communication can load software from one computer device or processor to another. For example, from one server or host computer to a hardware platform of a computer environment, or to other computer environments. Therefore, another medium used for transmitting software elements can also be used as the physical connection between local devices, such as light waves, radio waves, electromagnetic waves, etc., which can be propagated through cables, optical cables or air. Physical media used for carrier waves, such as cables, wireless connections or optical cables, can also be considered as media carrying software. As used herein, unless the tangible "storage" medium is limited, other terms representing computer or machine "readable medium" refer to the medium involved in the execution of any instruction by the processor.

The present disclosure uses specific words to describe the embodiments of the present disclosure. Such as "the first/second embodiment", "one embodiment", and/or "some embodiments" mean a certain feature, structure or characteristic related to at least one embodiment of the present disclosure. Therefore, it should be emphasized and noted that "one embodiment" or "an embodiment" or "an alternative embodiment" mentioned in different places in the specification do not necessarily mean the same embodiment. In addition, some features, structures, or characteristics in one or more embodiments of the present disclosure may be combined appropriately.

In addition, those skilled in the art can understand that various aspects of the present disclosure can be illustrated and described through several patentable categories or situations, including any new and useful processes, machines, products, substances or any combination thereof, or any new and useful improvements to them. Accordingly, all aspects of the present disclosure can be completely executed by hardware, software (including firmware, resident software, microcode, etc.), or a combination of hardware and software. The above hardware or software can be referred to as "data block," "module," "engine," "unit," "component," or "system." Furthermore, aspects of the present disclosure may be embodied as a computer product located in one or more computer-readable media, and the product includes computer-readable program code.

Unless otherwise defined, all terms (including technical and scientific terms) used herein have the same meanings as those commonly understood by those of ordinary skill in the art to which the present disclosure belongs. It should also be understood that terms such as those defined in general dictionaries should be interpreted as having meanings consistent with their meanings in the context of related art, and should not be interpreted in an idealized or extremely formal sense unless explicitly defined here.

The above is the description of the present disclosure, and should not be considered as a limitation thereof. Although several exemplary embodiments of the present invention have been described, those skilled in the art will readily understand that many modifications can be made to the exemplary embodiments without departing from the novel teaching and advantages of the present disclosure. Therefore, all these modifications are intended to be included within the scope of the present disclosure as defined by the claims. It should be understood that the above is the description of the present disclosure, and should not be regarded as limited to the specific embodiments disclosed, and modifications to the disclosed embodiments and other embodiments are intended to be included within the scope of the appended claims. The present disclosure is defined by the claims and their equivalents.

The present application claims priority of Chinese Patent Application No.201911167852.9, filed on November 25, 2019 and entitled "Medical Data Management Method, Medical Data Management Device and System," the entire disclosure of which is incorporated herein by reference as part of this application.

## Claims

1. A medical data management method, executed by a first client, comprising:
receiving a processing scheme associated with the first client from a server;
receiving medical data corresponding to the processing scheme from a medical device associated with the first client; and
transmitting the medical data to the server.

2. The medical data management method according to claim 1, further comprising:
determining a reminding message according to the received processing scheme; and
outputting the reminding message.

3. The medical data management method according to claim 2, further comprising:
forwarding the reminding message to a third client associated with the first client.

4. The medical data management method according to claim 1, wherein the medical device comprises at least two of:
a lung function instrument;
a ventilator;
an oxygen generator;
a sleep screening instrument;
an atomizer; and
a non-invasive blood multi-parameter detector.

5. A medical data management method, executed by a second client, comprising:
receiving a processing scheme associated with a first client from a server;
receiving medical data associated with the first client from a medical device;
updating the processing scheme based on the received medical data; and
transmitting the updated processing scheme to the server.

6. The medical data management method according to claim 5, wherein the first client and the second client are associated with each other.

7. The medical data management method according to claim 5, wherein the second client is a client of a first-level hospital, and the updated processing scheme comprises:
a processing scheme for leaving hospital;
a processing scheme for staying in hospital; or
a processing scheme for transferring to a hospital with higher level.

8. The medical data management method according to claim 5, wherein the second client is a client of a second-level hospital, and the updated processing scheme comprises:
a processing scheme for transferring to a hospital with lower level; or
a processing scheme for staying in hospital.

9. A medical data management method, executed by a server, comprising:
receiving medical data from a medical device from a first client;
transmitting the medical data received from the first client to a second client;
receiving an updated processing scheme from the second client; and
transmitting the updated processing scheme to the first client.

10. A medical data management apparatus, comprising:
an input unit, configured to receive a processing scheme associated with the medical data management apparatus from a server;
a medical data receiving unit, configured to receive medical data corresponding to the processing scheme from a medical device associated with the medical data management apparatus; and
an output unit, configured to transmit the medical data to the server.

11. The medical data management apparatus according to claim 10, further comprising:
a reminding unit, configured to determine a reminding message according to the received processing scheme;
wherein the output unit is further configured to output the reminding message.

12. The medical data management apparatus according to claim 11, further comprising:
a forwarding unit, configured to forward the reminding message to a third client associated with the medical data management apparatus.

13. The medical data management apparatus according to claim 10, wherein the medical device comprises at least two of:
a lung function instrument;
a ventilator;
an oxygen generator;
a sleep screening instrument;
an atomizer; and
a non-invasive blood multi-parameter detector.

14. A medical data management apparatus, comprising:
an input unit, configured to receive a processing scheme associated with a first client from a server;
a medical data receiving unit, configured to receive medical data from a medical device associated with the medical data management apparatus;
an updating unit, configured to update the processing scheme based on the received medical data; and
an output unit, configured to transmit the updated processing scheme to the server.

15. The medical data management apparatus according to claim 14, wherein the first client and the medical data management apparatus are associated with each other.

16. The medical data management apparatus according to claim 14, wherein the medical data management apparatus is a client of a first-level hospital, and the updated processing scheme comprises:
a processing scheme for leaving hospital;
a processing scheme for staying in hospital; or
a processing scheme for transferring to a hospital with higher level.

17. The medical data management apparatus according to claim 14, wherein the medical data management apparatus is a client of a second-level hospital, and the updated processing scheme comprises:
a processing scheme for transferring to a hospital with lower level; or
a processing scheme for staying in hospital.

18. A medical data management server, comprising a receiving unit and a transmitting unit,
wherein the receiving unit is configured to receive, from a first client, medical data from a medical device;
the transmitting unit is configured to transmit the medical data received from the first client to a second client,
the receiving unit is further configured to receive an updated processing scheme based on the medical data from the second client; and
the transmitting unit is further configured to transmit the updated processing scheme to the first client.

19. A medical data management system, comprising:
a first client, configured to receive medical data from a medical device;
a second client, associated with the first client; and
a server, configured to store a processing scheme associated with the first client and medical data, transmit the processing scheme and the medical data to the second client, receive an updated processing scheme from the second client, and transmit the updated processing scheme to the first client.

20. The medical data management system according to claim 19, wherein the second client is a client of a first-level hospital or a client of a second-level hospital.

21. A medical data management method, applied to a user terminal, comprising:
binding a user corresponding to the user terminal with a doctor;
transmitting information of the user to a server;
acquiring, from the server, a treatment scheme for the user given by the doctor , wherein the treatment scheme comprises using at least one medical device for examination, and medical data generated by the at least one medical device is processed and recorded by the server; and
acquiring medical data associated with the user generated by the at least one medical device from the server and displaying the medical data.

22. The medical data management method according to claim 21, wherein the information of the user comprises:
personal information of the user; and
answer information of the user with respect to at least one screening questionnaire.

23. The medical data management method according to claim 21 or 22, further comprising:
receiving a referral notification from the server.

24. A medical data management method, applied to a doctor terminal, comprising:
acquiring information of a bound user from a server and displaying the information of the user;
receiving a first input from a doctor to generate a treatment scheme for the user and transmitting the treatment scheme to the server, wherein the treatment scheme comprises using at least one medical device for examination;
acquiring medical data associated with the user generated by the at least one medical device from the server and displaying the medical data; and
receiving a second input from the doctor to indicate a subsequent treatment mode for the user determined based on the acquired medical data, and transmitting the treatment mode to the server, wherein the treatment mode comprises leaving hospital for treatment, staying in hospital for treatment or transferring to a second-level hospital higher than a first-level hospital for treatment in a case where the doctor terminal is a client of the first-level hospital, and the treatment scheme comprises staying in hospital for treatment or transferring to the first-level hospital for treatment in a case where the doctor terminal is a client of the second-level hospital.

25. The medical data management method according to claim 24, further comprising:
acquiring identification information of the doctor terminal from the server, and displaying the identification information, for binding the doctor terminal with the user.

26. The medical data management method according to claim 24, wherein in a case where the treatment mode is related to transferring to another hospital for treatment, the method further comprises:
receiving referral related information input by the doctor, and transmitting the referral related information to the server, so that the server transmits a referral request to another doctor terminal.

27. The medical data management method according to claim 26, wherein the method further comprises:
receiving, from the server, a referral response returned by the other doctor terminal to the server, wherein the server transmits a referral notification to a user terminal of the user based on the referral response.

28. The medical data management method according to claim 24, wherein the information of the user comprises:
personal information of the user; and
answer information of the user with respect to at least one screening questionnaire.

29. A medical data management method, applied to a server, comprising:
acquiring information of a bound user from a user terminal and transmitting the information of the user to a doctor terminal;
receiving, from the doctor terminal, a treatment scheme for the user given by a doctor and transmitting the treatment scheme to the user terminal, wherein the treatment scheme comprises using at least one medical device for examination;
acquiring medical data associated with the user from the at least one medical device, processing and recording the medical data, and transmitting the medical data to the user terminal and the doctor terminal; and
receiving information indicating a subsequent treatment mode for the user determined based on the acquired medical data from the doctor terminal.

30. The medical data management method according to claim 29, further comprising:
receiving referral related information from the doctor terminal;
transmitting a referral request to another doctor terminal based on the referral related information; and
transmitting a referral notification to the user terminal in response to receiving a referral response from the another doctor terminal.
